Europäisches Patentamt

(19) European Patent Office

Office européen des brevets

(11) Numéro de publication : **0 317 417 B1**

# FASCICULE DE BREVET EUROPEEN

(12)

(45) Date de publication du fascicule du brevet :
**22.01.92 Bulletin 92/04**

(21) Numéro de dépôt : **88402856.4**

(22) Date de dépôt : **15.11.88**

(51) Int. Cl.$^5$ : **A61M 16/00,** G05D 7/06

(54) **Dispositif d'assistance respiratoire.**

(30) Priorité : **19.11.87 FR 8716000**

(43) Date de publication de la demande :
**24.05.89 Bulletin 89/21**

(45) Mention de la délivrance du brevet :
**22.01.92 Bulletin 92/04**

(84) Etats contractants désignés :
**DE ES FR GB IT NL SE**

(56) Documents cités :
**DE-A- 2 414 693**
**US-A- 3 848 806**
**US-A- 4 097 786**

(73) Titulaire : **L'AIR LIQUIDE, SOCIETE ANONYME**
**POUR L'ETUDE ET L'EXPLOITATION DES**
**PROCEDES GEORGES CLAUDE**
**75, Quai d'Orsay**
**F-75321 Paris Cédex 07 (FR)**

Titulaire : **INSTITUT NATIONAL DE LA SANTE**
**ET DE LA RECHERCHE MEDICALE (INSERM)**
**101, rue de Tolbiac**
**F-75654 Paris Cédex 13 (FR)**

(72) Inventeur : **Zalkin, Daniel**
**7, rue de Tournebride**
**F-78120 Rambouillet (FR)**
Inventeur : **Isabey, Daniel**
**19, rue Sandrin**
**F-94140 Alfortville (FR)**
Inventeur : **Brochard, Laurent**
**15, avenue Jacques Jezequel**
**F-92170 Vanves (FR)**
Inventeur : **Harf, Alain**
**23, avenue de la Dame Blanche**
**F-94120 Fontenay-sous-Bois (FR)**

(74) Mandataire : **Le Moenner, Gabriel et al**
**Societé l'Air Liquide Chef du Service Brevets**
**et Marques 75, Quai d'Orsay**
**F-75321 Paris Cédex 07 (FR)**

**Description**

La présente invention concerne un dispositif d'assistance respiratoire s'adressant à des malades ayant perdu une partie de leur autonomie respiratoire.

On connaît des dispositifs de ce type qui comprennent un générateur de gaz respiratoire incorporant des moyens de maintien, pendant une phase inspiratoire, dans un conduit d'alimentation débouchant à un orifice respiratoire associé à une valve expiratoire, d'une pression substantiellement constante en gaz respiratoire, et cela quel que soit le débit de ce gaz inspiratoire.

Généralement, le maintien d'une pression substantiellement constante dans le conduit d'alimentation aboutissant à l'orifice respiratoire est assuré par un moyen de mesure de la pression, qui est utilisé pour commander le débit de gaz respiratoire pendant la phase inspiratoire, elle-même initiée par un appel du patient lors d'un début d'inspiration et qui se termine par un refus du patient à une fin d'inspiration. A la suite de cette phase inspiratoire, une phase expiratoire généralement passive, permet au patient de vider ses poumons avant de réinitier une nouvelle phase respiratoire.

Les dispositifs connus de ce type sont essentiellement constitués de dispositifs de ventilation réalisant la fonction décrite ci-dessus à l'aide de moyens relativement complexes et coûteux. Cette complexité des moyens mis en oeuvre permet cependant le choix d'autres modes de ventilation.

La présente invention vise un dispositif d'assistance respiratoire exclusivement destiné à l'aide respiratoire, qui soit de réalisation simple et efficace, et particulièrement peu coûteux. Selon l'invention, dans un dispositif d'assistance respiratoire du genre rappelé ci-dessus, les moyens d'établissement d'une pression substantiellement constante comprennent un éjecteur du type à convergent/divergent avec admission d'air et tuyère injectrice axiale, débouchant directement dans le circuit d'alimentation équipé d'un capteur de débit commandant, via un ensemble électronique de gestion du signal, l'ouverture ou la fermeture d'une vanne montée entre une source de gaz sous pression et ladite tuyère axiale. Avantageusement l'éjecteur comporte une partie convergente suivie d'une partie de section constante formant un col allongé, lui-même débouchant dans un diffuseur divergent. Selon une forme particulière de réalisation, le col de l'éjecteur a un diamètre compris entre 10 mm et 14 mm et, également de préférence, l'angle d'ouverture du diffuseur divergent est compris entre 5° et 10° et de préférence de l'ordre de 7°.

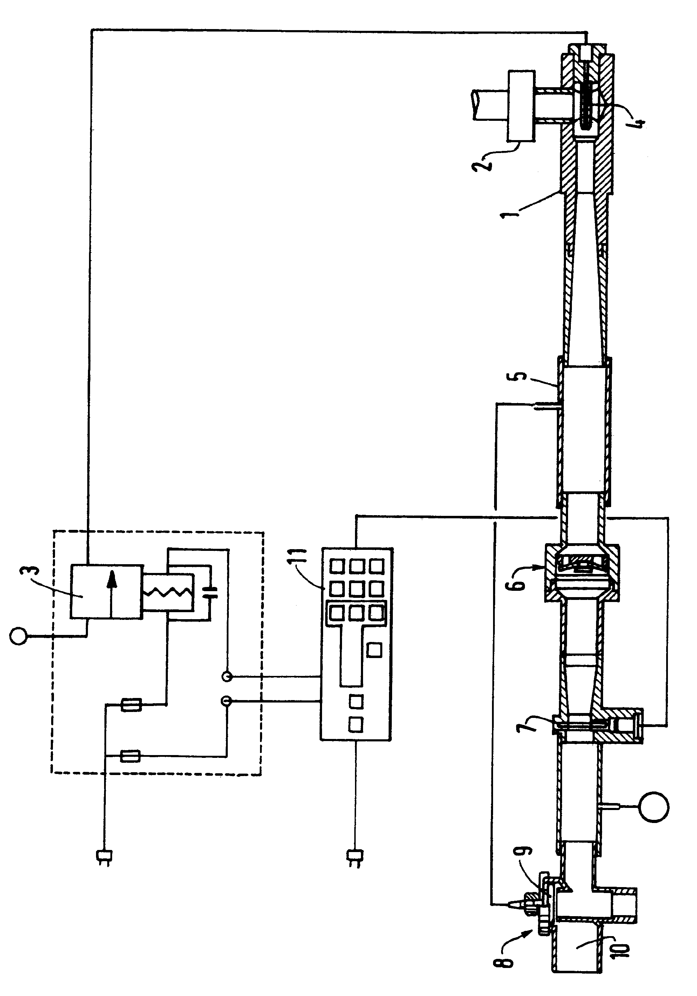

Les caractéristiques et avantages de l'invention ressortiront d'ailleurs de la description qui suit, à titre d'exemple, en référence au dessin annexé dont la figure unique représente schématiquement un dispositif selon l'invention.

Ce dispositif comporte :
— un éjecteur 1 muni, à l'entrée d'air, de filtres bactériologiques 2 et qui reçoit un débit d'air comprimé délivré à partir d'une source de pression d'environ 3,5 bars non représentée et autorisé par une électrovanne 3. Ce débit, défini par une tuyère 4 de l'éjecteur 1 et la pression disponible, génère dans le circuit aval une pression sensiblement constante quel que soit le débit total soutiré, ajustable entre 5 et 30 mbar selon le diamètre de la tuyère 4 que l'on peut faire varier par choix d'une tuyère parmi un jeu de tuyères de différents diamètres, ou de toute autre façon,
— une canalisation de liaison 5 raccordant la sortie de l'éjecteur 1 à un clapet anti-retour 6 sans résistance ; ce clapet a pour rôle d'empêcher le retour du gaz expiré afin d'en éviter la réinhalation.
— un capteur de débit à fil chaud 7 permettant de mesurer le débit de gaz le traversant et utilisé comme organe de mise en marche et en arrêt de l'électrovanne 3.
— une valve expiratoire 8 munie d'un ballonnet gonflable 9 qui obture l'orifice expiratoire 10 lorsque l'éjecteur débite et l'ouvre à l'arrêt de débit de l'éjecteur 1, la pression de gonflage du ballonnet étant prise en amont du clapet anti-retour.
— un ensemble électronique 11 de question du signal émis par le capteur de débit assure l'ouverture de l'électrovanne 3 lorsqu'un seuil de débit minimum est franchi, débit généré par l'appel de l'aspiration du patient, et la fermeture de l'électrovanne 3 lorsque le débit inspiré par le patient retombe en dessous d'un second seuil de débit. Ces deux seuils sont ajustables pour une bonne adaptation à la demande du patient.

L'expérience a montré que l'éjecteur 1 permet de façon simple et efficace, le maintien, à l'aval, d'une pression substantiellement constante, dès lors que cet éjecteur est alimenté en gaz. Cet éjecteur peut avoir des formes assez variables, mais de bons résultats ont été obtenus avec un diffuseur divergent de l'ordre de 7° avec un col de tuyère de l'ordre de 0,8 à 1,2 mm.

Les domaines d'application de l'invention sont les suivants :

Contrairement aux ventilateurs aux multiples modes de ventilation qui s'adressent généralement à des malades intubés ou trachéotomisés, l'invention s'adresse à des malades que l'on relie au dispositif par un simple masque enfermant le nez et la bouche, un masque nasal ou un embout buccal.

La source de pression peut être le circuit de distribution d'air médical en milieu hospitalier ou un petit compresseur muni des filtres adaptés à la production d'air respirable.

La population de malades redevables de cette technique est principalement représentée par les post-opérés et les insuffisants respiratoires chroniques.

## Revendications

1. Dispositif d'assistance respiratoire, du genre comprenant un générateur de gaz respiratoire incorporant des moyens d'établissement, pendant une phase inspiratoire, dans un conduit d'alimentation (5) débouchant à un orifice inspiratoire (10) associé à une valve expiratoire (8), d'une pression substantiellement constante d'un gaz respiratoire, quel que soit le débit du gaz inspiratoire, caractérisé en ce que les moyens d'établissement d'une pression substantiellement constante comprennent un éjecteur du type à convergent-divergent (1) avec admission d'air (2) et tuyère injectrice axiale (4) débouchant dans ledit conduit d'alimentation (5), équipé d'un capteur de débit (7) commandant, via un ensemble électronique de gestion de signal (11) l'ouverture ou la fermeture d'une vanne (3) montée entre une source de gaz sous pression et ladite tuyère axiale (4).

2. Dispositif d'assistance respiratoire selon la revendication 1, caractérisé en ce que l'éjecteur présente une partie convergente suivie d'un col de diamètre constant et d'un diffuseur divergent.

3. Dispositif d'assistance respiratoire selon la revendication 2, caractérisé en ce que le col a un diamètre de l'ordre de 10 à 14 mm.

4. Dispositif d'assistance respiratoire selon la revendication 1, caractérisé en ce que l'angle du diffuseur divergent est compris entre 5 et 10° et de préférence de l'ordre de 7°.

5. Dispositif d'assistance respiratoire selon la revendication 1, caractérisé en ce que le débit de gaz respiratoire est introduit dans l'éjecteur par l'intermédiaire d'un tuyère interchangeable en fonction de la pression d'assistance souhaitée, et dont le diamètre de conduit est compris entre 0,8 et 1,2 mm.

## Patentansprüche

1. Atmungshilfsgerät mit einem Generator für Atmungsgas, der Einrichtungsmittel für einen im wesentlichen konstanten Druck eines Beatmungsgases während einer Einatmungsphase in einer Versorgungsleitung (5) aufweist, die in einer Einatmungsöffnung (10) mündet, welche einem Ausatmungsventil (8) zugeordnet ist, unabhängig vom Durchsatz des Einatmungsgases, dadurch gekennzeichnet, daß die Einrichtungsmittel für einen im wesentlichen konstanten Druck einen Ejektor vom konvergent-divergenten Typ (1) mit Luftzfuhr (2) und axialer Einpritzdüse (4) aufweisen, welche in der Versogungsleitung (5) mündet, wobei die Versorgungsleigung (5) mit einem Meßfühler (7) für den Durchsatz ausgestattet ist, der über eine elektronische Anordnung (11) für die Steuerung eines Signals das Öffnen oder Schließen eines Ventils (3) steuert, welches zwischen einer Quelle für unter Druck stehendes Gas und der axialen Düse (4) angebracht ist.

2. Atmungshilfsvorrichtung nach Anspruch 1, dadurch gekennzeichnet, daß der Ejektor einen konvergenten Teil aufweist, der von einem Hals mit konstantem Durchmesser und einem divergenten Diffusor gefolgt ist.

3. Beatmungshilfsvorrichtung nach Anspruch 2, dadurch gekennzeichnet, daß der Hals einen Durchmesser in der Größenordnung von 10 bis 14 mm hat.

4. Beatmungshilfsvorrichtung nach Anspruch 1, dadurch gekennzeichnet, daß der Winkel des divergenten Diffusors zwischen 5 und 10° liegt und vorzugsweise in der Größenordnung von 7° beträgt.

5. Beatmungshilfsvorrichtung nach Anspruch 1, dadurch gekennzeichnet, daß der Durchsatz des Beatmungsgases in den Ejektor mittels austauschbaren Düse in Funktion des gewünschten Hilfsdruckes eingeführt wird un daß dessen Leitungsdurchmesser zwischen 0,8 und 1,2 mm beträgt.

## Claims

1. Respiratory aid device, of the type comprising a respiratory gas generator incorporating means for providing, during an inspiratory phase, in a supply pipe (5) opening at an inspiratory aperture (10) associated with an expiratory valve (8), a substantially constant pressure of a respiratory gas, whatever the flow of the inspiratory gas, **characterised in that** the means for providing a substantially constant pressure comprise an ejector of the converging-diverging type (1) having an air admission (2) and an axial injector nozzle (4) opening into the said supply pipe (5), the said ejector being provided with a flow sensor (7) controlling, by way of an electronic signal management assembly (11), the opening or closing of a valve (3) mounted between a pressurised gas source and the said axial nozzle (4).

2. Respiratory aid device according to claim 1, **characterised in that** the ejector has a converging part followed by a neck of constant diameter and a diverging diffuser.

3. Respiratory aid device according to claim 2, **characterised in that** the neck has a diameter of the order of 10 to 14 mm.

4. Respiratory aid device according to claim 1, **characterised in that** the angle of the diverging diffuser is between 5 and 10° and preferably of the order of 7°.

5. Respiratory aid device according to claim 1, **characterised in that** the respiratory gas flow is introduced into the ejector by means of a nozzle which can be interchanged in dependence upon the desired respiratory aid pressure, and the pipe diameter of which is between 0.8 and 1.2 mm.